# EUROPEAN PATENT APPLICATION

(11) **EP 4 830 773 A1**
(43) Date of publication of application: **28.10.2026**
(21) Application number: 26164226.8
(22) Date of filing: 12.03.2026
(51) Int. Cl.: A61N 5/10, G21K 1/04

(54) **MULTI-LEAF COLLIMATORS AND RADIOTHERAPY MACHINES INCLUDING THE SAME**

(30) Priority: 21.04.2025 US 202519184110
(71) Applicant: Varian Medical Systems, Inc., Palo Alto, CA 94304 (US)
(72) Inventor: SCOLLAY, Stuart, Palo Alto, CA, 94304 (US); MCWILLIAMS, Christopher, Palo Alto, CA, 94304 (US); GEE, Andrew, Palo Alto, CA, 94304 (US)
(74) Representative: HKW Intellectual Property PartG mbB

(57) **Abstract**

A multilayer multi-leaf collimator (MLC) includes a first leaf box configured to hold a plurality of first beam blocking leaves, wherein the plurality of first beam blocking leaves are configured to move relative to one another and relative to the first leaf box, each of the plurality of first beam blocking leaves having a first end and a second end; and a first stop coupled to the first leaf box, the first stop configured to permit the first ends of the plurality of first beam blocking leaves to move up to a set distance in a first direction past the first stop, the set distance being less than lengths of the plurality of first beam blocking leaves.

## Description

### TECHNICAL FIELD

One or more example embodiments relate to multi-leaf collimators (MLCs) and radiotherapy treatment delivery machines including the same.

### BACKGROUND

Multi-leaf collimators (MLCs) are used in radiotherapy treatment delivery machines to support radiation therapy treatments such as intensity-modulated radiation therapy (IMRT) and others. Conventional MLCs include sets of beam blocking leaves arranged in two opposing banks. In operation, each of the individual beam blocking leaves is positioned to block a portion of a radiation beam passing through the volume occupied by the leaf. The combined positioning of all beam blocking leaves defines one or many apertures through which the unblocked radiation beam passes, and the aperture(s) define(s) the shape of the radiation beam directed to a treatment field at an isocenter.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

At least one example embodiment provides a multilayer multi-leaf collimator (MLC) includes a first leaf box configured to hold a plurality of first beam blocking leaves, wherein the plurality of first beam blocking leaves are configured to move relative to one another and relative to the first leaf box, each of the plurality of first beam blocking leaves having a first end and a second end; and a first stop coupled to the first leaf box, the first stop configured to permit the first ends of the plurality of first beam blocking leaves to move up to a set distance in a first direction past the first stop, the set distance being less than lengths of the plurality of first beam blocking leaves.

At least one example embodiment provides a radiation therapy treatment machine comprising a radiation source configured to emit a radiation beam; and a multi-leaf collimator to block portions of the radiation beam to define a treatment field for the radiation therapy treatment machine, the multi-leaf collimator including a first leaf box configured to hold a plurality of first beam blocking leaves, wherein the plurality of first beam blocking leaves are configured to move relative to one another and relative to the first leaf box, each of the plurality of first beam blocking leaves having a first end and a second end, and a first stop coupled to the first leaf box, the first stop configured to permit the first ends of the plurality of first beam blocking leaves to move up to a set distance in a first direction, the set distance being less than lengths of the plurality of first beam blocking leaves.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIG. 1 is a side perspective view of a multi-leaf collimator (MLC) according to example embodiments.
FIG. 2A and FIG. 2B are front perspective views of the MLC shown in FIG. 1
FIG. 3 is a side view of the MLC shown in FIG. 1.
FIG. 4 illustrates the MLC shown in FIG. 1 with the leaf boxes removed.
FIG. 5 illustrates the MLC shown in FIG. 1 with the leaf boxes removed.
FIG. 6 is a perspective view of a leaf box according to example embodiments.
FIG. 7 illustrates another view of the leaf box shown in FIG. 6.
FIG. 8A illustrates a proximal leaf according to example embodiments.
FIG. 8B illustrates a distal leaf according to example embodiments.
FIG. 9A illustrates a portion of the MLC shown in FIG. 1 with a first stop removed.
FIG. 9B illustrates a portion of the MLC shown in FIG. 1 with a first stop removed.
FIG. 10 illustrates a front view of a proximal stop according to example embodiments.
FIG. 11 illustrates a front view of a distal stop according to example embodiments.
FIGS. 12A-12B illustrate cross-sectional views of the first stop and the third stop engaging a proximal leaf and a distal leaf, respectively, according to example embodiments.
FIG. 13A illustrates a stop according to at least one example embodiment.
FIG. 13B illustrates a leaf according to at least one example embodiment.
FIG. 14 illustrates an example of a radiation therapy treatment machine, according to example embodiments;
FIG. 15 is a block diagram illustrating an example embodiment of the controller 220 shown in FIG. 14.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

While operations in one or more figures may be presented as occurring in series and in a certain order, example embodiments are not so limited. The operations may be performed in a different order and/or in parallel, and they may also be performed in an iterative manner.

Conventional radiotherapy devices include a primary collimation system upstream from a multi-leaf collimator (MLC). The primary collimator is a fixed aperture that initially shapes the radiation beam emerging from the linear accelerator, providing a basic level of beam shaping and limiting the maximum field size.

Downstream of the primary collimator, movable x and y jaws provide further refinement of the beam shape. These jaws may be adjusted independently to create a rectangular or square field of varying dimensions.

With dual-layer stacked MLCs, jaws may be omitted to save vertical space in the treatment head. However, with the reduction in pre-collimation prior to the MLC, there is the potential for field leakage to the side of the leaf banks. Additional outboard or side shielding leaves or components are used to mitigate and/or prevent this field leakage.

Additionally, in a conventional stacked MLC with movable leaf boxes, accurately placing shielding components may be relatively difficult since the outboard shielding components must clear the leaf boxes as the leaf boxes move.

One or more example embodiments provide multilayer MLCs including outboard shielding components (e.g., edge defining and/or scatter-blocking leaves) that span or extend across a radiation therapy treatment field from a guide feature (also referred to as a guide groove or guide component) in one leaf box to a matching guide feature in an opposing leaf box. Unlike pairs of beam blocking leaves, which moveably extend to a central portion of a radiation therapy treatment field, each of the outboard shielding components extends from within an outer edge of one leaf box across the treatment field to within a corresponding outer edge of the opposing leaf box. By spanning between the two leaf boxes, the placement of the outboard shielding components may have the same or substantially the same tolerances as the other beam blocking leaves of the MLC.

Outboard shielding components may include edge defining leaves and/or scatter-blocking leaves. The edge defining leaves define and shape the opposing edges of a radiation therapy treatment field for a patient during treatment. The scatter-blocking leaves block or reduce the scattered radiation that reaches healthy tissue of the patient. Outboard shielding components may also be referred to as outboard shielding elements, out of field shielding elements, out of field shielding components, boundary shielding elements or boundary shielding components.

As described herein, beam blocking leaves, edge defining leaves and/or scatter-blocking leaves may be referred to more generally as shielding elements or components. Shielding elements may be formed of one or more high density materials, such as tungsten.

In accordance with one or more example embodiments, the MLC includes a first stop to prevent the shielding components from completely sliding out of the leaf boxes.

In one or more example embodiments, the leaves include a raised surface that rigidly contact the first stop, which may attach to the front of the MLC assembly. The first stop is intricately designed to follow the orientation angle of the leaves as they translate along the MLC assembly. The first stop makes no contact with a leaf until the raised surface that prevents further forward travel and prevents the leaf from falling out of the MLC assembly.

In other example embodiments, the leaves have a sharpy raised surface or a lower surface followed by a sharply higher surface, e.g., a notch. A first stop with flexible beams is attached to the front of the MLC assembly. The flexible beams are axially rigid. The flexible beams have surface contact with the leaves and allow leaf travel until reaching the raised surface or the notch. Upon reaching the raised surface or notch, the flexible beams on the first stop will prevent further forward motion and prevent the leaf from falling out of the MLC assembly.

In one or more example embodiments, the first stop may be a bracket that is placed across a leaf box.

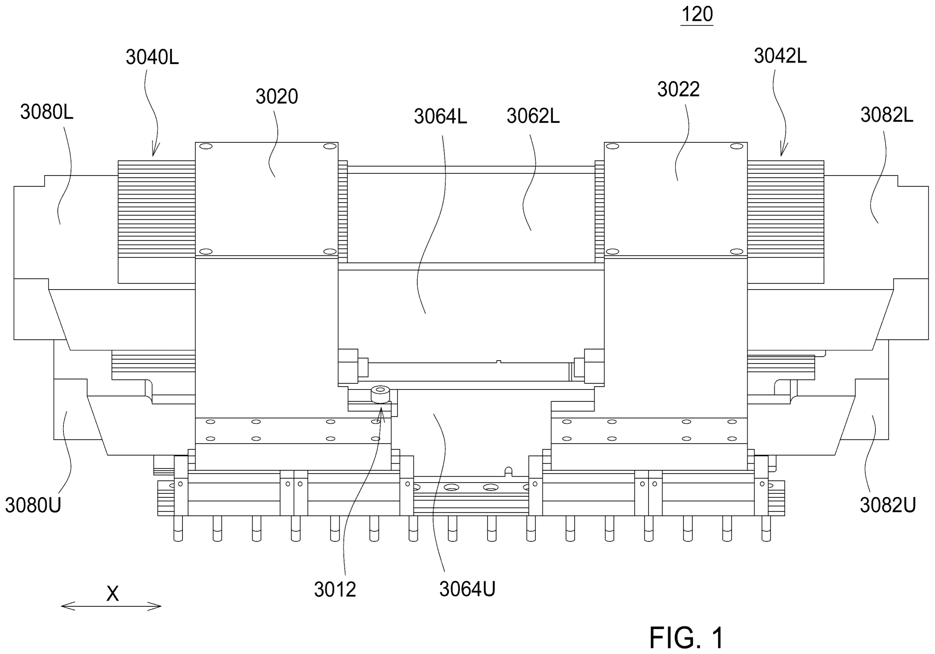

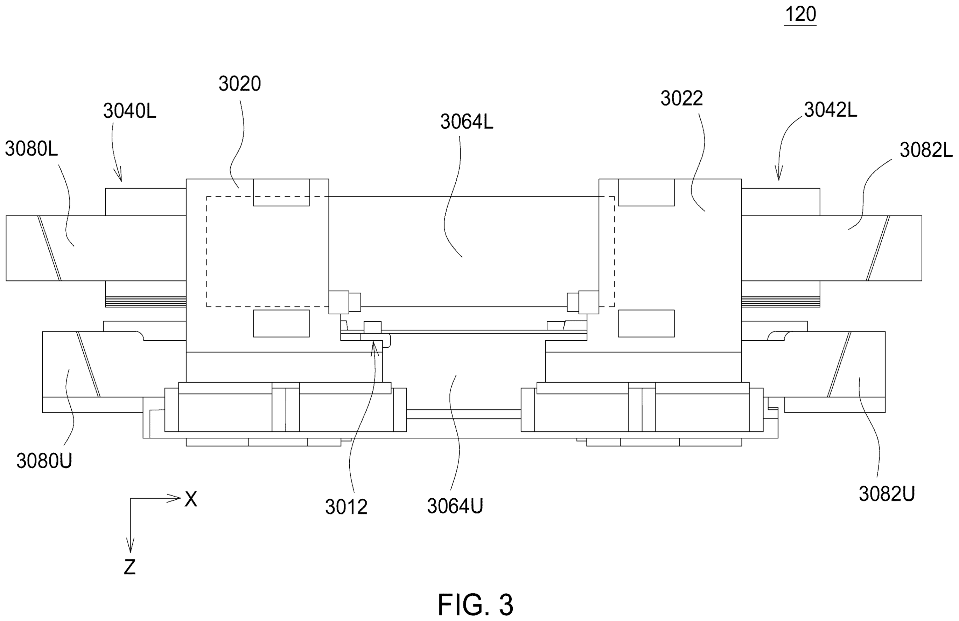

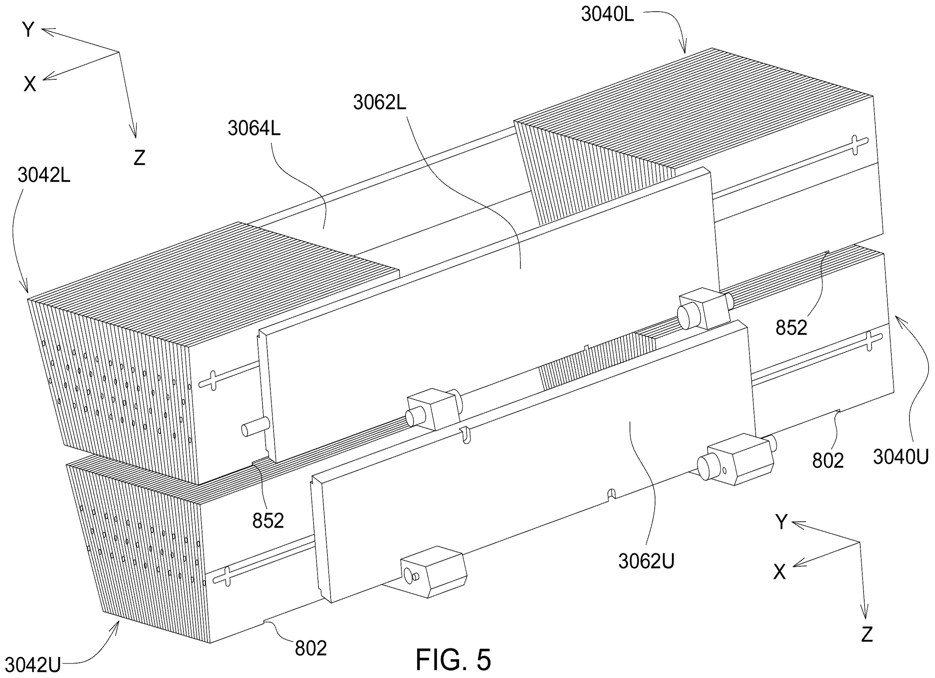

Example embodiments of MLCs, and components thereof, will now be described in detail with regard to FIGS. 1-15.

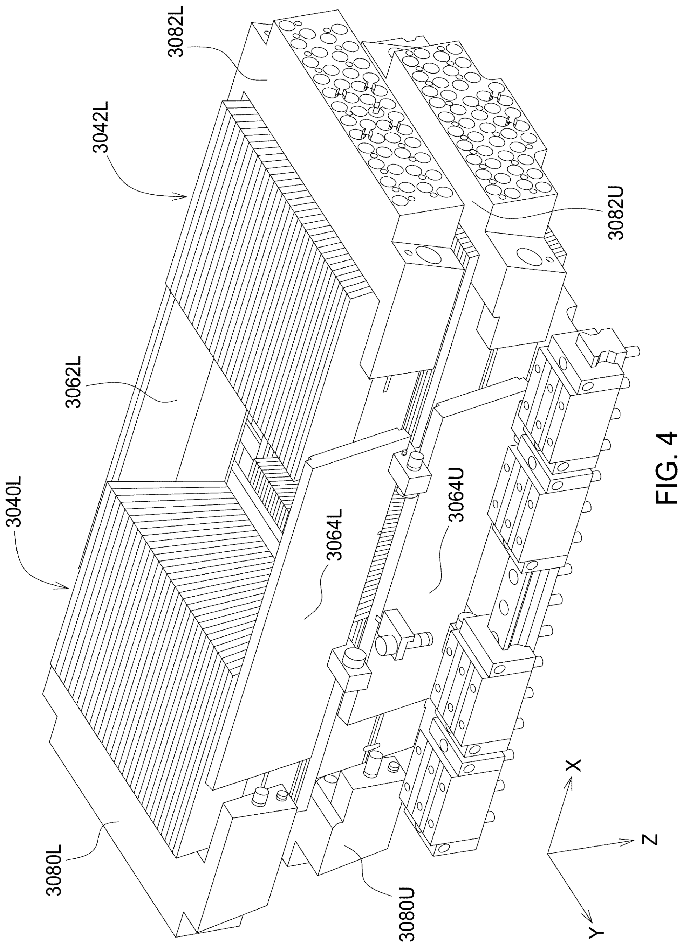

FIGS. 1, 2A, 2B and 3 illustrate various views of a MLC according to example embodiments. FIGS. 4-5 illustrate the MLC shown in FIG. 1 with the leaf boxes removed.

Referring to FIGS. 1-5, the MLC 120 includes a first leaf box 3020 and a second leaf box 3022 arranged opposite one another. The first leaf box 3020 is configured to move in a first direction (X-direction). The second leaf box 3022 is also configured to move in the first direction such that the first leaf box 3020 and the second leaf box 3022 move linearly toward and away from one another. The first leaf box 3020 and the second leaf box 3022 may be moved by respective carriage motors (not shown). Because carriage motors are generally known, a detailed discussion is omitted.

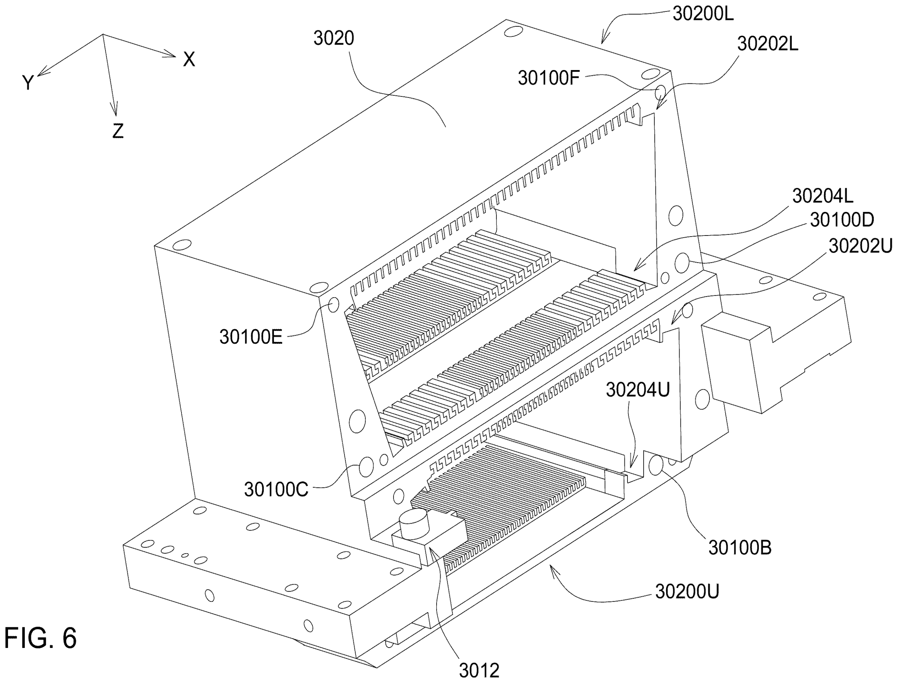

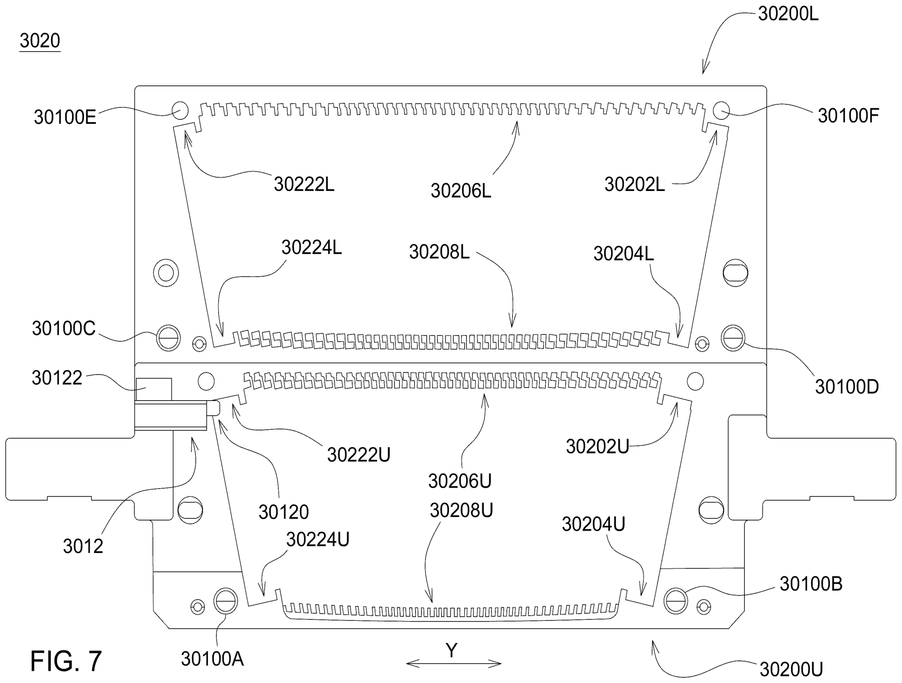

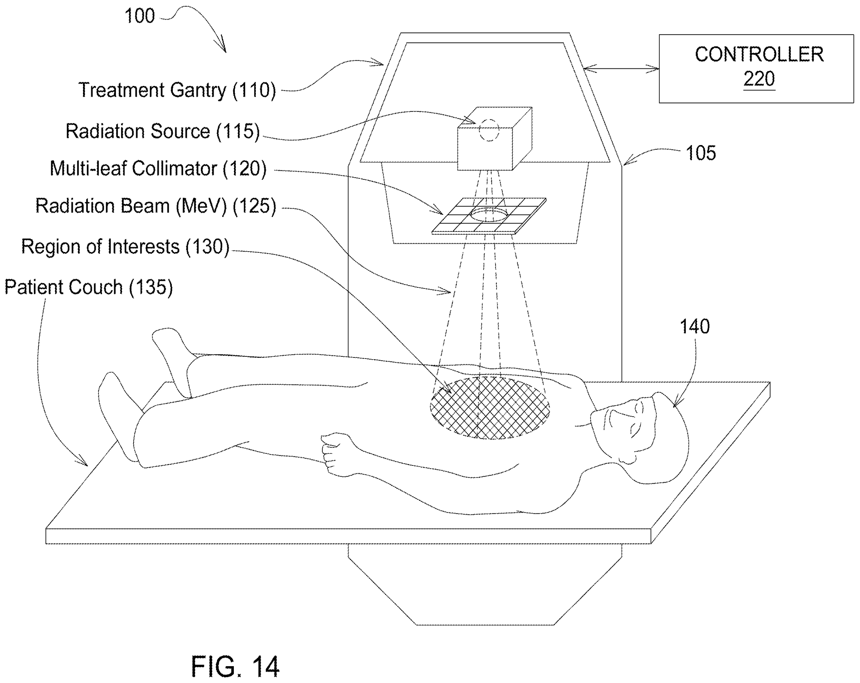

The first leaf box 3020 includes a proximal portion (30200U in FIGS. 6 and 7 discussed below) and a distal portion (30200L in FIGS. 6 and 7 discussed below). The terms "proximal" and "distal" are used herein to describe positioning of elements of the MLC 120 relative to a radiation source (e.g., a radiation source 115, shown in FIG. 14).

In the proximal portion, the first leaf box 3020 is configured to hold a plurality of first proximal beam blocking leaves 3040U (also referred to as upper beam blocking leaves) arranged adjacent to one another (side-by-side) in a first linear array or row. Each of the first proximal beam blocking leaves 3040U is configured to move independently in the first direction relative to one another and relative to the first leaf box 3020. Each of the plurality of first proximal beam blocking leaves 3040U may be moved/driven independently by drive assembly 3080U, which includes one or more drive motors.

In the distal portion, the first leaf box 3020 is configured to hold a plurality of first distal beam blocking leaves 3040L arranged adjacent to one another (side-by-side) in a linear array or row below the plurality of first proximal beam blocking leaves 3040U. Each of the first distal beam blocking leaves 3040L is also configured to move independently in the first direction relative to one another, relative to the first leaf box 3020 and relative to the plurality of first proximal beam blocking leaves 3040U. The plurality of first distal beam blocking leaves 3040L may be moved/driven independently by drive assembly 3080L, which includes one or more drive motors.

The second leaf box 3022 also includes a proximal portion and a distal portion. In the proximal portion, the second leaf box 3022 is configured to hold a plurality of second proximal beam blocking leaves 3042U arranged adjacent to one another (side-by-side) in a second linear array or row. Each of the second proximal beam blocking leaves 3042U is configured to move independently relative to one another and relative to the second leaf box 3022. The second proximal beam blocking leaves 3042U may also be configured to move relative to the plurality of first proximal beam blocking leaves 3040U. The plurality of second proximal beam blocking leaves 3042U may be moved/driven independently by drive assembly 3082U, which includes one or more drive motors.

In the distal portion, the second leaf box 3022 is configured to hold a plurality of second distal beam blocking leaves 3042L arranged adjacent to one another (side-by-side) in a linear array or row below the plurality of second proximal beam blocking leaves 3042U. Each of the second distal beam blocking leaves 3042L are configured to move independently relative to one another, relative to the plurality of second proximal beam blocking leaves 3042U, relative to the first and second leaf boxes 3020 and 3022, and relative to the beam blocking leaves 3040U and 3040L. The plurality of second distal beam blocking leaves 3042L may be moved/driven independently by drive assembly 3082L, which includes one or more drive motors.

According to one or more example embodiments, the first proximal beam blocking leaves 3040U and the second proximal beam blocking leaves 3042U are arranged in pairs (e.g., one first proximal beam blocking leaf and one second proximal beam blocking leaf), wherein each pair of proximal beam blocking leaves may move independently (e.g., based on a radiation therapy treatment plan) to block or allow a radiation beam to pass through to the patient.

Similarly, the first distal beam blocking leaves 3040L and the second distal beam blocking leaves 3042L are arranged in pairs (e.g., one first distal beam blocking leaf and one second distal beam blocking leaf), wherein each pair of distal beam blocking leaves may move independently (e.g., based on a radiation therapy treatment plan) to block or allow a radiation beam to pass through to the patient.

Still referring to FIGS. 1-5, the MLC 120 further includes edge defining leaves 3062L and 3064L extending between the distal portions of the first leaf box 3020 and the second leaf box 3022.

As shown in FIGS. 4-5, the distal blocking leaves 3040L and 3042L include a transition portion 852 that prevents the proximal blocking leaves 3040U and 3042U from sliding out of the boxes 3020 and 3022, respectively. Distal blocking leaves have a similar transition portion.

Returning to FIGS. 1-5, the edge defining leaves 3062L and 3064L are arranged at respective ends of the rows of distal beam blocking leaves 3040L and 3042L to define respective edges of a radiation therapy treatment field.

The MLC 120 further includes scatter-blocking leaves 3062U and 3064U extending between the proximal portions of the first leaf box 3020 and the second leaf box 3022.

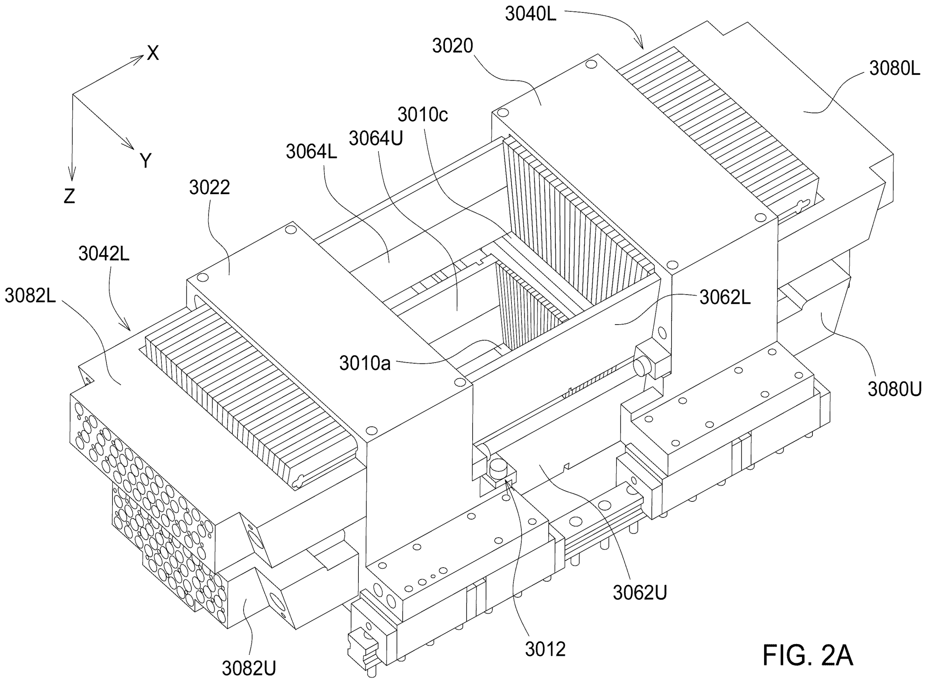

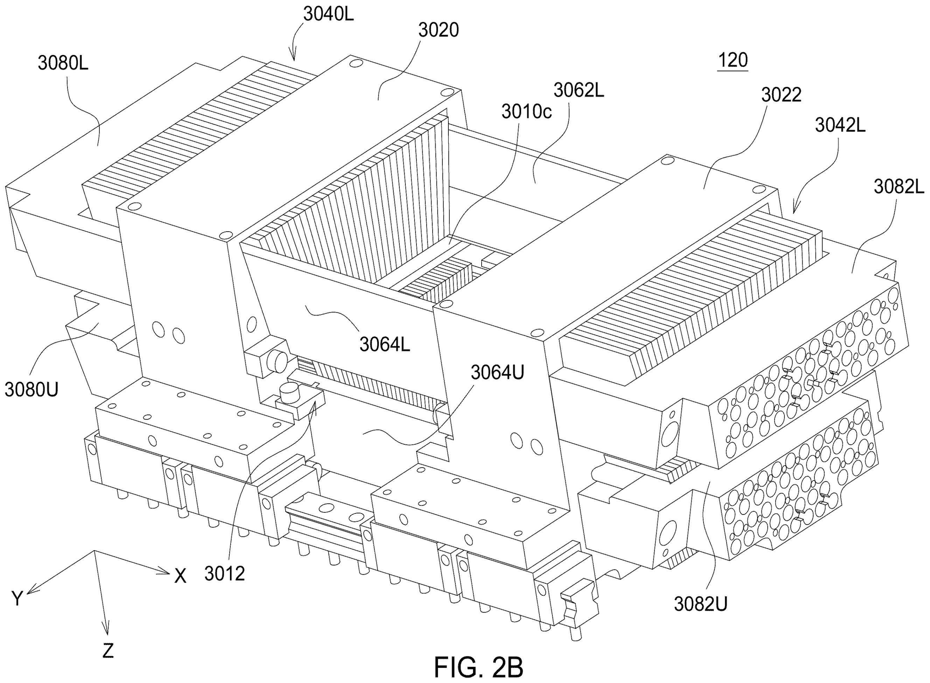

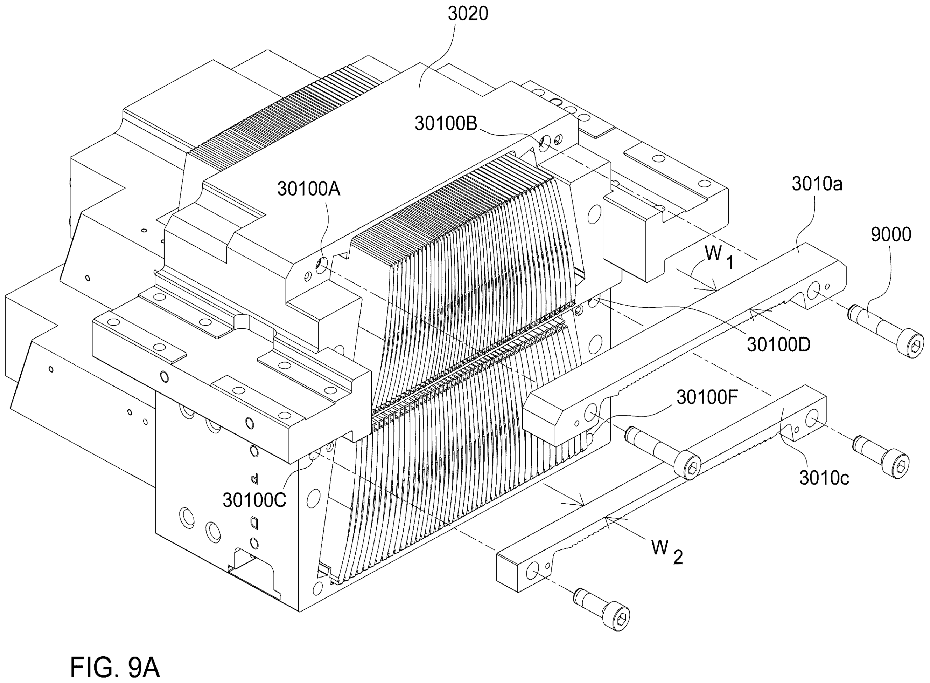

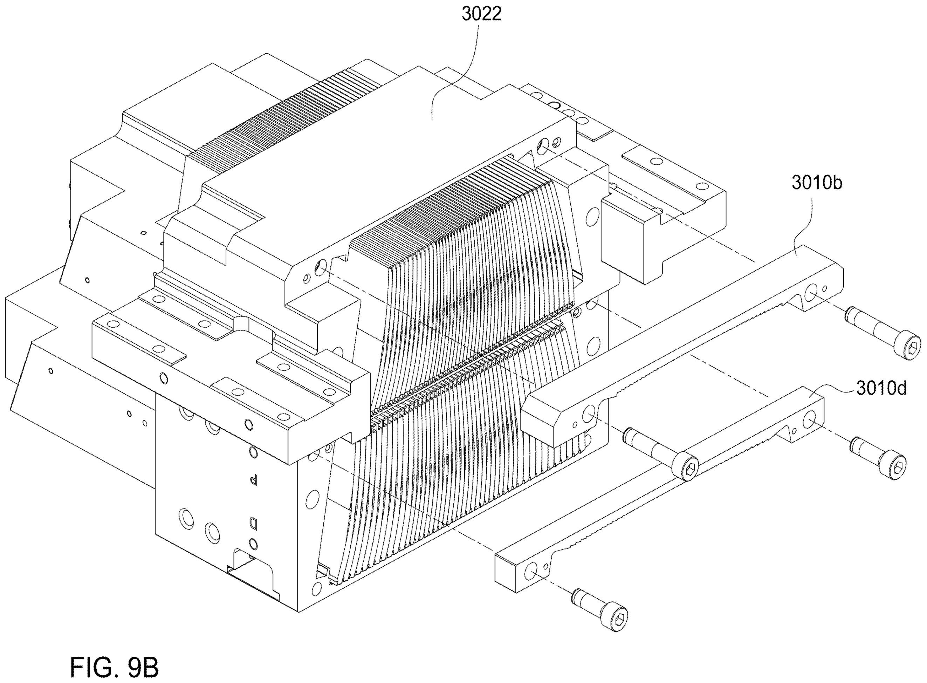

While not shown in FIG. 1, a first stop 3010a (shown in FIGS. 2A and 9A) engages with a proximal portion of the first leaf box 3020. Similarly, a second stop 3010b (shown in FIG. 9B) engages with a proximal portion of the second leaf box 3022. As shown in FIG. 2A, stops 3010a and 3010c also engage in proximal and distal portions, respectively, of the first leaf box 3020. As described and shown in more detail below, for example, a portion of the first stop 3010a engages with transition portions 802 of the leaves 3040U while a portion of the third stop 3010c engages with transition portions 852 of the leaves 3040L to prevent the leaves 3040U and 3040L from sliding out. Similarly, the second stop engages with transition portions 802 of the leaves 3042U while a portion of a fourth stop engages with transition portions 852 of the leaves 3042L to prevent the leaves 3042U and 3042L from sliding out. In the example embodiments shown in FIGS. 1-5, the transition portions 802 and 852 are transitions from a first surface to a second surface. However, example embodiments are not limited thereto.

FIGS. 6-7 illustrate the first leaf box 3020 of FIG. 1 in more detail. For the sake of brevity, only the first leaf box 3020 will be described. It should be understood, however, that the second leaf box 3022 may be the same or substantially the same as the first leaf box 3020. When implemented in a MLC according to one or more example embodiments, the second leaf box 3022 may be the same as the first leaf box 3020, but rotated 180 degrees about a vertical axis.

Referring to FIGS. 6-7, the first leaf box 3020 includes a proximal portion 30200U and a distal portion 30200L.

The proximal portion 30200U includes first upper proximal outer groove 30204U and first lower proximal outer groove 30202U to hold the scatter-blocking leaf 3062U in place to block scatter radiation and to guide the scatter-blocking leaf 3062U as the scatter-blocking leaf 3062U moves relative to the first leaf box 3020. At an opposite side of the proximal portion 30200U in the Y-direction, the proximal portion 30200U includes second upper proximal outer groove 30224U and second lower proximal outer groove 30222U to hold the scatter-blocking leaf 3064U in place to block scatter radiation as the scatter-blocking leaf 3064U moves along with the first leaf box 3020.

The proximal portion 30200U of the first leaf box 3020 further includes a plurality of upper proximal inner grooves 30208U between the upper proximal outer grooves 30204U and 30224U, and a plurality of lower proximal inner grooves 30206U between the lower proximal outer grooves 30202U and 30222U.

The pluralities of upper and lower proximal inner grooves 30208U and 30206U hold and guide the plurality of first proximal beam blocking leaves 3040U during movement thereof.

The distal portion 30200L includes first upper distal outer groove 30204L and first lower distal outer groove 30202L to hold the edge-defining leaf 3062L at an edge of a radiation therapy treatment field as the edge-defining leaf 3062L moves relative to the first leaf box 3020. At an opposite side in the Y-direction, the distal portion 30200L includes second upper distal outer groove 30224L and second lower distal outer groove 30222L to hold the edge-defining leaf 3064L at an edge of a radiation therapy treatment field as the edge defining leaf 3064L moves along with the first leaf box 3020.

The distal portion 30200L of the first leaf box 3020 further includes a plurality of upper distal inner grooves 30208L between the upper distal outer grooves 30204L and 30224L, and a plurality of lower distal inner grooves 30206L between the lower distal outer grooves 30202L and 30222L.

The pluralities of upper and lower distal inner grooves 30208L and 30206L hold and guide the plurality of first distal beam blocking leaves 3040L during movement thereof.

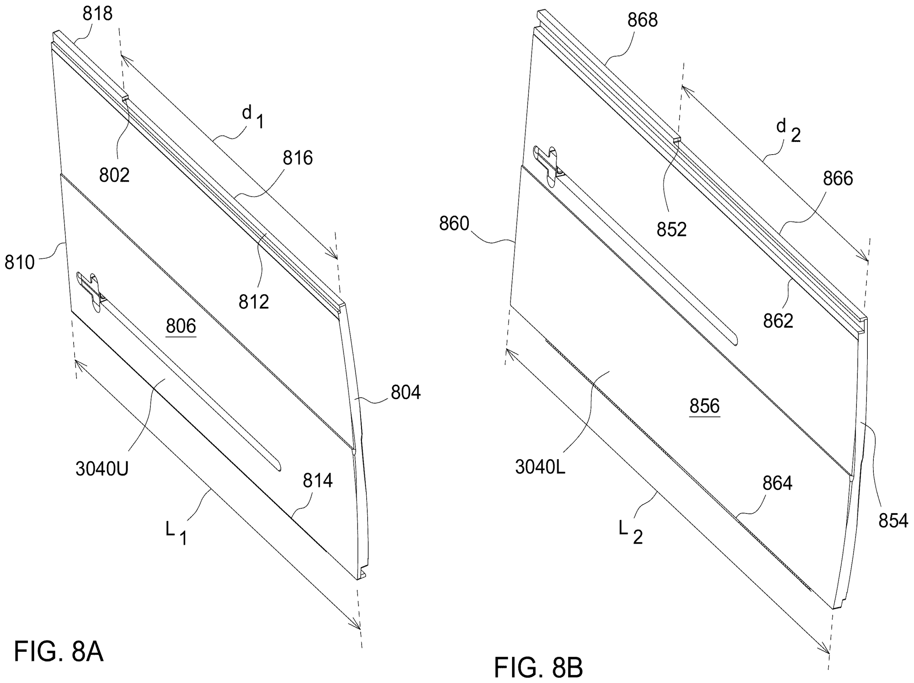

FIG. 8A illustrates a proximal leaf according to example embodiments. FIG. 8B illustrates a distal leaf according to example embodiments.

As shown in FIG. 8A, the proximal leaf 3040U includes the transition portion 802 to restrict movement of the proximal leaf 3040U relative to the first leaf box 3020 during operation. That is, for example, the first stop 3010a engages with the first leaf box 3020 and the transition portion 802 such that the proximal leaf 3040U is able to move toward the second leaf box 3022 (i.e., in a first direction) until the first stop 3010a engages the transition portion 802. The proximal leaf 3040U has a length L₁ The transition portion 802 occurs at a distance d₁ from the first end 804. The first end 804 faces the leaf box 3022 and is exposed to the treatment field.

A main body 806 of the proximal leaf 3040U includes a first end 804 and a second end 810. A first side 812 and a second side 814 of the main body 806 extend between the first end 804 and the second end 810. The first side 812 and the second side 814 are on opposing sides of the main body 806.

The main body 806 is a flat rectangular shaped beam blocking material. As discussed in more detail later, the transition portion 802 allows the movement of the proximal leaf 3040U to be fixed or stationary relative to a leaf box.

The transition portion 802 is on the first side 812. The first side 812 includes a first surface 816 and a second surface 818. The transition portion 802 connects the first surface 816 and the second surface 818. Thus, the first surface 816 extends from the first end 804 to the transition portion 802 and the second surface 818 extends from the second end 810 to the transition portion 802. As a result of the transition portion 802, the second surface 818 is a raised surface relative to the first surface 816.

The first stop 3010a is configured to permit the first end 804 of the proximal leaf 3040U to move up to a set distance in the first direction past the first stop 3010a by contacting the transition portion 802 when the first end 804 reaches the set distance. The set distance may be a difference between the distance d₁ of the main body 806 and a width w₁ of the first stop 3010a.

It should be understood that the proximal leaves 3042U have the same structure as shown in FIG. 8A. Thus, the second stop 3010b is configured to permit the first end 804 of the proximal leaf 3042U to move up to a set distance in the first direction past the second stop 3010b by contacting the transition portion when the first end reaches the set distance.

As shown in FIG. 8B, the distal leaf 3040L includes a transition portion 852 to restrict movement of the distal leaf 3040L relative to the first leaf box 3020 during operation. That is, for example, the third stop 3010c engages with the first leaf box 3020 and the transition portion 852 such that the distal leaf 3040L is able to move toward the second leaf box 3022 (i.e., in a first direction) until the third stop 3010c engages the transition portion 852. The distal leaf 3040L has a length L₂ of a main body 856. In some example embodiments, the length L₂ is less than the length L₁. The transition portion 852 occurs at a distance d₂ from the first end 854. The first end 854 faces the leaf box 3020 and is exposed to the treatment field. In some example embodiments, the distance d₂ is different than the distance d₁. The distances d₁ and d₂ and lengths L₁ and L₂ may be set such that the proximal leaves 3040U and the distal leaves 3040L (in combination with leaf box 3020) may travel to close a distance to the leaf box 3022 (e.g., to zero) with the leaf box 3022 stationary.

The main body 856 includes a first end 854 and a second end 860. The main body 806 is a flat rectangular shaped beam blocking material. As discussed in more detail later, the transition portion 802 allows the movement of the proximal leaf 3040U to be fixed or stationary relative to a leaf box.

A first side 862 and a second side 814 of the main body 856 extend between the first end 854 and the second end 860. The first side 862 and the second side 864 are on opposing sides of the main body 856. The transition portion 852 is on the first side 862. The first side 862 includes a first surface 866 and a second surface 868. The transition portion 852 connects the first surface 866 and the second surface 868. Thus, the first surface 866 extends from the first end 854 to the transition portion 852 and the second surface 868 extends from the second end 860 to the transition portion 852. As a result of the transition portion 852, the second surface 868 is a raised surface relative to the first surface 866.

The third stop 3010c is configured to permit the first end 854 of the distal leaf 3040L to move up to a set distance in the first direction past the third stop 3010c by contacting the transition portion 852 when the first end 854 reaches the set distance. The set distance may be a difference between the distance d₁ of the main body 856 and a width w₂ of the third stop 3010c.

The first ends 804 and 854 define at least a portion of the treatment field.

While the transition portions 802 and 852 are shown as including normal (or substantially normal) transitions from the first surface to the second surface, it should be understood that the transition portion may be linear and extends at a non-normal angle relative to the first surface. In other example embodiments, the transition portion may be concave or convex.

As shown in FIG. 9A, the first stop 3010a engages with the first leaf box 3020 above the second upper proximal outer groove 30222U by inserting fasteners 9000 (e.g., screws and/or bolts) into bores 30100A and 30100B. In addition, the third stop 3010c engages with the first leaf box 3020 above the second upper distal outer groove 30222L by inserting fasteners into bores 30100C and 30100D (see, e.g., FIG.9A).

Similarly, as shown in FIG. 9B, the second stop 3010b and the fourth stop 3010d engage with the second leaf box 3022 in the same or similar manner as the first stop 3010a and the third stop 3010c engage with the first leaf box 3020. Therefore, a description thereof is omitted.

Still referring to FIGS. 6-7, a retaining bracket assembly 3012, which is secured by a screw and washer assembly 30122, has a protruding portion 30120 that extends in the Y-direction and engages with a notch to restrict movement of the scatter-blocking leaf 3064U relative to the first leaf box 3020 during operation. That is, for example, the retaining bracket assembly 3012 engages with the notch such that the scatter-blocking leaf 3064U moves along with the first leaf box 3020 in the same or substantially the same manner as the edge defining leaf 3064L. Similar to the edge defining leaf 3064L, at the second leaf box 3022, the scatter-blocking leaf 3064U is allowed to slide and move relative to the second leaf box 3022 as the first leaf box 3020 is moved.

In one example, the screw portion of the screw and washer assembly 30122 may be a socket head cap screw.

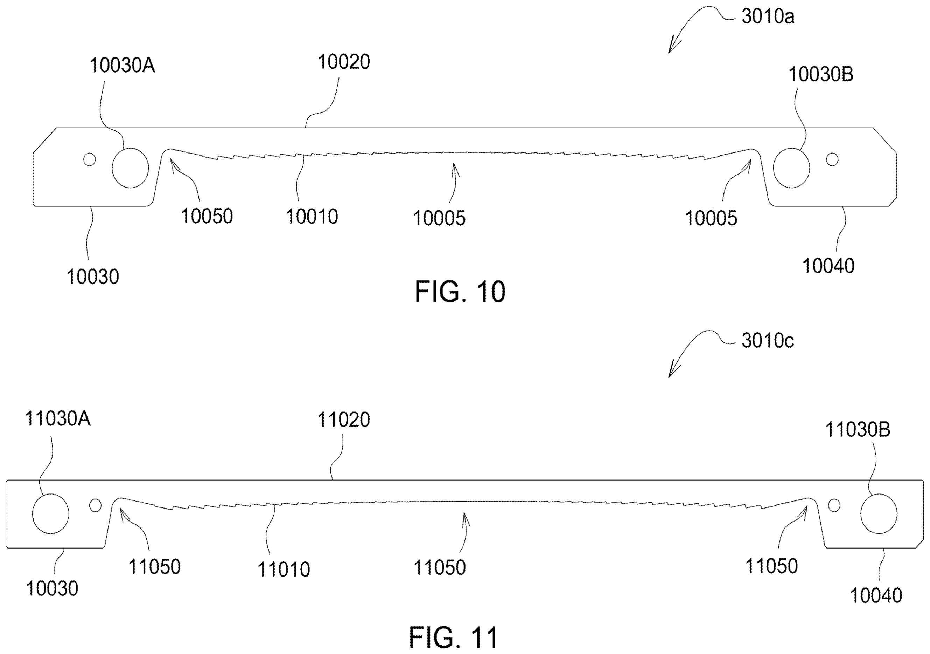

FIG. 10 illustrates a front view (i.e., from the treatment field) of the first stop 3010a. It should be understood that the second stop 3010b (another proximal stop) has the same structure and, thus, is not described in further detail.

As shown, the first stop 3010a includes two outer grooves 10005 and a plurality of inner grooves 10010. The two outer grooves 10005 are configured to receive the leaves 3064U and 3062U, respectively. The first stop 3010a includes a top surface 10020 that extends in a longitudinal direction of the first stop 3010a, end portions 10030, 10040 and a receiving side 10050 between the two end portions 10030, 10040. The receiving side 10050 opposes a portion of the top surface 10020 and includes the outer grooves 10005 and inner grooves 10010. Each inner groove 10010 corresponds to an inner groove 30208U of the leaf box 3020. Thus, an inner groove 10010 and the corresponding inner groove 30208U form a pair of grooves that guide a proximal leaf 3040U that is inserted into the corresponding inner groover 30208U. Each inner groove 10010 is set at a position relative to the top surface 10020 of the first stop 3010a such that the surface 816 of the corresponding proximal leaf 3040U either does not contact the groove 10010 or slides along the groove 10010. In addition, the inner groove 10010 engages the transition portion 802 when the first end 804 moves the set distance from the first stop 3010a. The engagement of the groove 10010 with the transition portion 802 of the corresponding proximal leaf 3040U prevents further movement of the proximal leaf 3040U towards the second leaf box 3022.

The first stop 3010a further includes bores 10030A and 10030B at opposing ends of the first stop 3010a to receive the fasteners 9000.

FIG. 11 illustrates a front view (i.e., from the treatment field) of the third stop 3010c. It should be understood that the fourth stop 3010d (another distal stop) has the same structure and, thus, is not described in further detail.

As shown, the third stop 3010c includes two outer grooves 11005 and a plurality of inner grooves 11010. The two outer grooves 11005 are configured to receive the leaves 3064L and 3062L, respectively. The third stop 3010c includes a top surface 11020 that extends in a longitudinal direction of the third stop 3010c, end portions 11030, 11040 and a receiving side 11050 between the two end portions 11030, 11040. The receiving side 11050 opposes a portion of the top surface 11020 and includes the outer grooves 11005 and inner grooves 11010. Each inner groove 11010 corresponds to an inner groove 30208U of the leaf box 3020. Thus, an inner groove 11010 and the corresponding inner groove 30208U form a pair of grooves that guide a distal leaf 3040L that is inserted into the corresponding inner groover 30208U. Each inner groove 11010 is set at a position relative to the top surface 11020 of the third stop 3010c such that the surface 866 of the corresponding distal leaf 3040L either does not contact the groove 11010 or slides along the groove 11010. In addition, the inner groove 11010 engages the transition portion 852 when the first end 854 moves the set distance from the third stop 3010c. The engagement of the groove 11010 with the transition portion 852 of the corresponding distal leaf 3040L prevents further movement of the distal leaf 3040L towards the second leaf box 3022.

The third stop 3010c further includes bores 11030A and 11030B at opposing ends of the third stop 3010c to receive the fasteners 9000.

While the stops 3010a-3010d are shown positioned on top portions of the leaves, it should be understood that notches may be located on bottom portions of the leaves and the stops 3010a-3010d may be positioned to correspond to the locations of the notches (e.g., if the notches are on a bottom portion of the leaves, the corresponding stop responsible for stopping the leaves is at the bottom portion of the leaves).

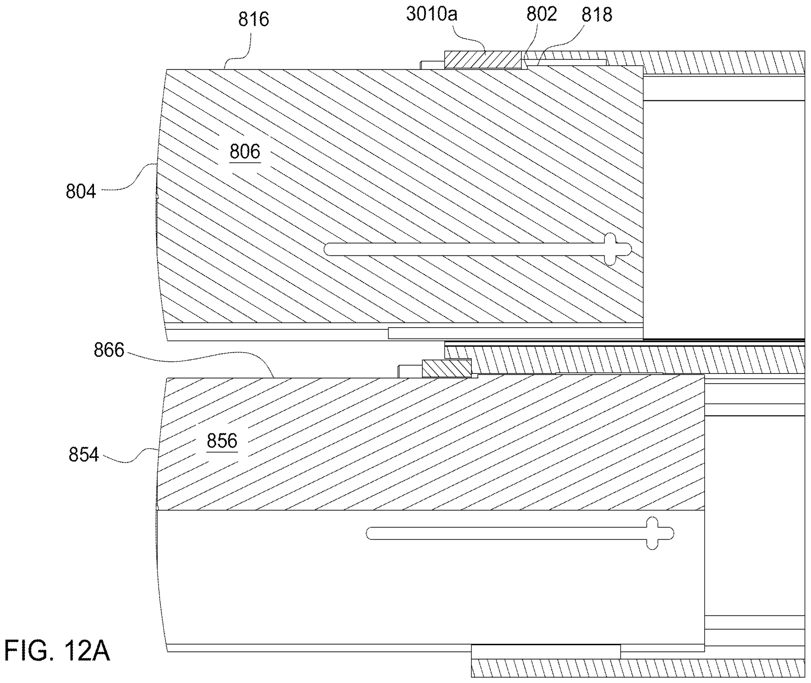

FIGS. 12A-12B illustrates cross-sectional views of the first stop and the third stop engaging a proximal leaf and a distal leaf, respectively, according to example embodiments. More specifically, FIG. 12A illustrates the first stop 3010a engaging/contacting the transition portion 802 and the third stop 3010c engaging/contacting the transition portion 852.

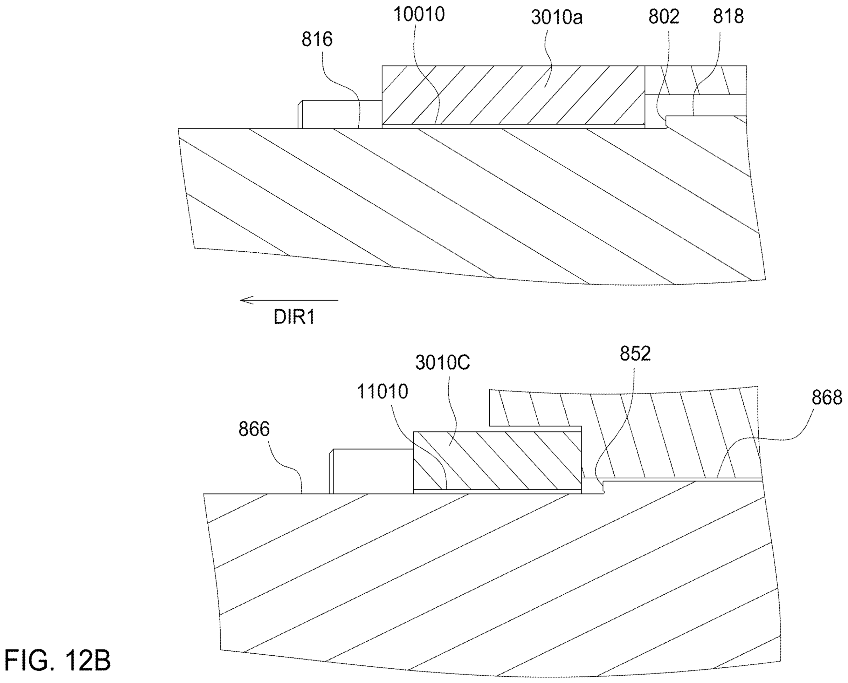

FIG. 12B illustrates a zoomed-in view of the first stop 3010a engaging/contacting the transition portion 802 and the third stop 3010c engaging/contacting the transition portion 852. As shown in FIG. 12B, the transition portion 802 is a surface that connects the surfaces 816 and 818 by extending at a normal angle relative to the surface 816 and at a normal angle relative to surface 818. Similarly, the transition portion 852 is a surface that connects the surfaces 866 and 868 by extending at a normal angle relative to the surface 866 and at a normal angle relative to surface 868. The grooves 10010 and 11010 prevent the leaves 3040U and 3040L from moving in the direction DIR1 along the x-axis during to the engagement with the transition portions 802 and 852.

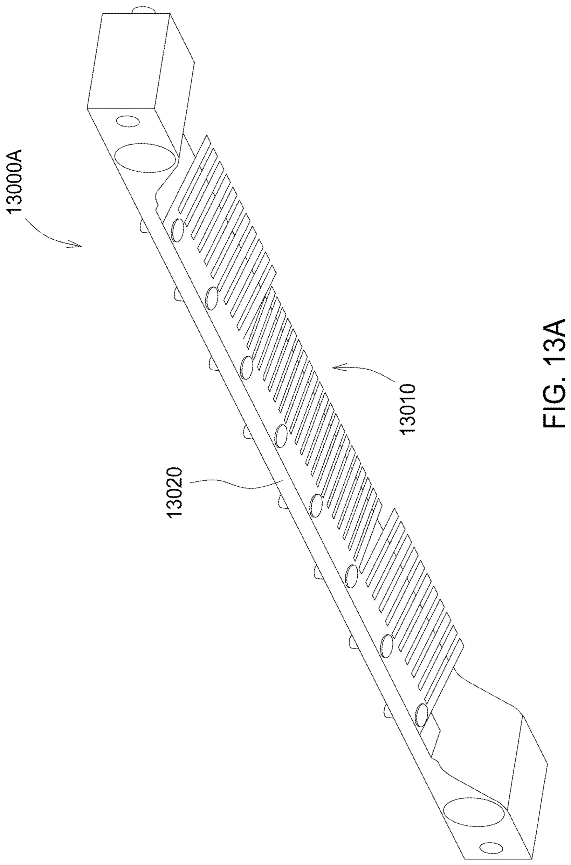

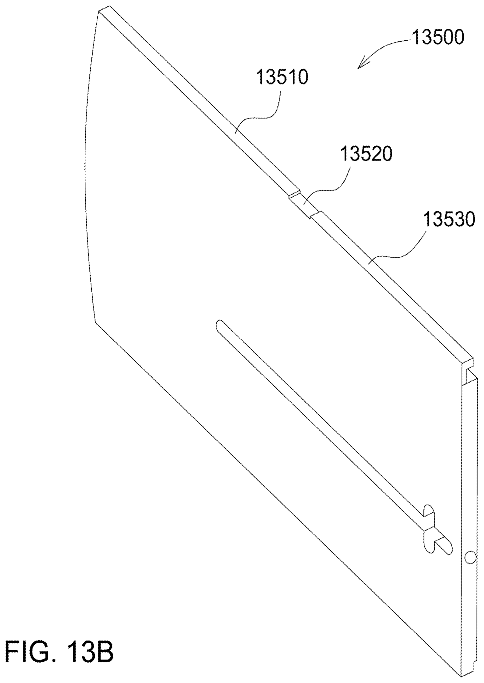

FIG. 13A illustrates a stop according to at least one example embodiment. FIG. 13B illustrates a leaf according to at least one example embodiment. A stop 13000A has a similar structure as the third stop 3010c. Similarly, a leaf 13500 has a similar structure as the leaf 3040L. Thus, only differences will be described for the sake of brevity.

As shown in FIG. 13B, the leaf 13500 has a raised surface 13510 followed by a lower surface 13520 and then followed by another raised surface 13530. Thus, the surface 13520 forms part of a notch between the raised surfaces 13510 and 13530. The stop 13000A includes elongated flexible beams 13010 attached to a front side 13020 of the stop 13000A. The flexible beams 13010 are axially rigid. The flexible beams 13010 have surface contact with the leaves 13500 and allow leaf travel until reaching the notch. Upon reaching the notch, the flexible beams 13010 will prevent further forward motion and prevent the leaf from falling out of the MLC assembly. In other embodiments, the stop 13000A may be used with the proximal leaves 3040U/3042U and/or the distal leaves 3040L/3042L.

FIG. 14 illustrates a radiation therapy treatment machine including the MLC 120, according to example embodiments.

Referring to FIG. 14, the radiation therapy treatment machine 100 includes a patient couch 135, on which a patient 140 may be positioned so that the region of interest 130 is properly located within the radiation beam 125. The treatment gantry 110 includes a radiation source 115 and the MLC 120. The radiation source 115 directs the radiation beam 125, through the MLC 120, and towards the region of interest (ROI) 130. Individual beam blocking leaves and outboard shielding elements of the MLC 120 are arranged to block portions of the radiation beam 125 that fall outside the region of interest 130 to define the radiation therapy treatment field.

In some example embodiments, the patient couch 135 includes multiple movable parts (not illustrated) to position the patient couch 135 under the treatment gantry 110 and next to, within, or partially within the treatment unit 105. Furthermore, in some example embodiments, the treatment gantry 110 may include movable parts that enable the treatment gantry 110 to be rotated about the patient couch 135 or otherwise moved relative to the region of interest 130. Movement of the treatment gantry 110 or the patient couch 135 may cause the region of interest 130 to move with respect to the radiation source 115 and the MLC 120. Changes in the relative position of the region of interest 130 may cause the shape and size of the region of interest to vary, which require individual beam blocking leaves of the MLC 120 to be moved to block different portions of the radiation beam 125 and modify the radiation therapy treatment field.

The radiation therapy treatment machine 100 is in two-way communication with a controller 220 configured to control operation of one or more components of the radiation therapy treatment machine 100 shown in FIG. 14 concurrently, simultaneously, separately, individually, etc.

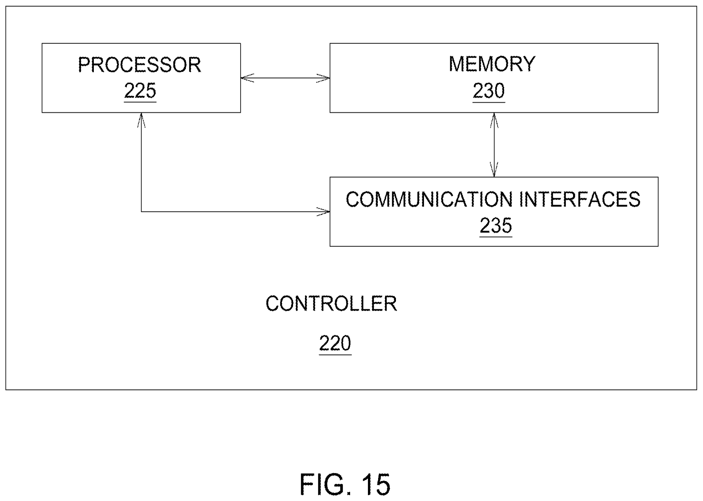

FIG. 15 is a block diagram illustrating an example embodiment of the controller 220 shown in FIG. 14.

Referring to FIG. 15, the controller 220 includes at least one processor 225, at least one memory 230, and at least one communication interface 235. The at least one memory 230 may be configured to store instructions that may be executed by the at least one processor 225 to cause the radiation therapy treatment machine 100 to perform one or more functions such as executing a radiotherapy procedure, control the MLC 120, etc.

As will be appreciated, depending on the implementation of the controller 220, the controller 220 may include additional components. However, it is not necessary that all of these generally conventional components be shown in order to illustrate example embodiments. For example purposes, the controller 220 will be discussed with regard to the processor 225. However, it should be understood that the controller 220 may include one or more processors or other processing circuitry, such as one or more Application Specific Integrated Circuits (ASICs).

The memory 230 may be a computer readable storage medium that generally includes a random access memory (RAM), read only memory (ROM), and/or a permanent mass storage device, such as a disk drive. The memory 230 may also store an operating system and any other routines/modules/applications for providing the functionalities of the controller 220 to be executed by the processor 225. These software components may also be loaded from a separate computer readable storage medium into the memory 230 using a drive mechanism (not shown). Such separate computer readable storage medium may include a disc, tape, DVD/CD-ROM drive, memory card, or other like computer readable storage medium (not shown). In some example embodiments, software components may be loaded into the memory 230 via one of the various communication interfaces 235, rather than via a computer readable storage medium.

The processor 225 or other processing circuitry may be configured to carry out instructions of a computer program by performing the arithmetical, logical, and input/output operations of the system. Instructions may be provided to the processor 225 by the memory 230.

The various communication interfaces 235 may be wired or wireless and may include components that interface the processor 225 with the other input/output components and/or one or more communications networks. As will be understood, the various communication interfaces 235 and programs stored in the memory 230 to set forth the special purpose functionalities of the controller 220 may vary depending on the implementation of MLC 120 and/or the radiation therapy treatment machine 100.

The various communication interfaces 235 may also include one or more user input devices (e.g., a keyboard, a keypad, a mouse, or the like) and user output devices (e.g., a display, a speaker, or the like).

### NON-LIMITING ILLUSTRATIVE EMBODIMENTS

According to a first aspect, a multi-leaf collimator (MLC) to define a treatment field for a radiation therapy treatment machine is suggested, the multi-leaf collimator comprising: a first leaf box configured to hold a plurality of first beam blocking leaves, wherein the plurality of first beam blocking leaves are configured to move relative to one another and relative to the first leaf box, each of the plurality of first beam blocking leaves having a first end and a second end; and a first stop coupled to the first leaf box, the first stop configured to permit the first ends of the plurality of first beam blocking leaves to move up to a set distance in a first direction past the first stop, the set distance being less than lengths of the plurality of first beam blocking leaves.

According to an embodiment, at least one of the plurality of first beam blocking leaves includes a first side and a second side opposing the first side, the first side and the second side extending between the first end and the second end, the first side being configured to contact the first stop when the at least one first beam blocking leaf is moving in the first direction.

According to a further embodiment, the first side includes a first surface and a second surface, the first surface being a greater distance from the second side than the second surface.

According to a further embodiment, the at least one first beam blocking leaf includes a transition portion from the first surface to the second surface and the first stop is configured to contact the transition portion when the first end of the at least one first beam blocking leaf reaches the set distance.

According to a further embodiment, the MLC includes the transition portion extends at angle normal or substantially normal to the first surface.

According to a further embodiment, the first surface and the second surface define a notch therebetween.

According to a further embodiment, the first stop includes at least one elongated member configured to engage the notch.

According to a further embodiment, the at least one elongated member is configured to contact the first surface as the at least one first beam blocking leaf moves.

According to a further embodiment, the MLC further comprises a second leaf box configured to hold a plurality of second beam blocking leaves, wherein the plurality of second beam blocking leaves are configured to move relative to one another and relative to the second leaf box, each of the plurality of second beam blocking leaves having a first end and a second end; and a second stop coupled to the second leaf box, the second stop configured to permit the first ends of the plurality of second beam blocking leaves to move up to the set distance past the second stop in a second direction.

According to a further embodiment, the first ends of the plurality of first beam block leaves and the first ends of the plurality of second beam block leaves define at least a portion of the treatment field.

According to a second aspect, a radiation therapy treatment machine is suggested, the radiation therapy treatment machine comprising a radiation source configured to emit a radiation beam; and a multi-leaf collimator to block portions of the radiation beam to define a treatment field for the radiation therapy treatment machine, the multi-leaf collimator including a first leaf box configured to hold a plurality of first beam blocking leaves, wherein the plurality of first beam blocking leaves are configured to move relative to one another and relative to the first leaf box, each of the plurality of first beam blocking leaves having a first end and a second end, and a first stop coupled to the first leaf box, the first stop configured to permit the first ends of the plurality of first beam blocking leaves to move up to a set distance in a first direction, the set distance being less than lengths of the plurality of first beam blocking leaves.

According to a further embodiment, at least one of the plurality of first beam blocking leaves includes a first side and a second side opposing the first side, the first side and the second side extending between the first end and the second end, the first side being configured to contact the first stop when the at least one first beam blocking leaf is moving in the first direction.

According to a further embodiment, the first side includes a first surface and a second surface, the first surface being a greater distance from the second side than the second surface.

According to a further embodiment, the at least one first beam blocking leaf includes a transition portion from the first surface to the second surface and the first stop is configured to contact the transition portion when the first end of the at least one first beam blocking leaf reaches the set distance.

According to a further embodiment, the transition portion extends at angle normal or substantially normal to the first surface.

According to a further embodiment, the first surface and the second surface define a notch therebetween.

According to a further embodiment, the first stop includes at least one elongated member configured to engage the notch.

According to a further embodiment, the at least one elongated member is configured to contact the first surface as the at least one first beam blocking leaf moves.

According to a further embodiment, the radiation therapy treatment machine includes a second leaf box configured to hold a plurality of second beam blocking leaves, wherein the plurality of second beam blocking leaves are configured to move relative to one another and relative to the second leaf box, each of the plurality of second beam blocking leaves having a first end and a second end; and a second stop coupled to the second leaf box, the second stop configured to permit the first ends of the plurality of second beam blocking leaves to move up to the set distance past the second stop in a second direction.

According to a further embodiment the first ends of the plurality of first beam block leaves and the first ends of the plurality of second beam block leaves define at least a portion of the treatment field.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Graphics Processing Units (GPUs), one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors, one or more GPUs, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

## Claims

1. A multi-leaf collimator, MLC, to define a treatment field for a radiation therapy treatment machine, the multi-leaf collimator comprising:
a first leaf box configured to hold a plurality of first beam blocking leaves, wherein the plurality of first beam blocking leaves are configured to move relative to one another and relative to the first leaf box, each of the plurality of first beam blocking leaves having a first end and a second end; and
a first stop coupled to the first leaf box, the first stop configured to permit the first ends of the plurality of first beam blocking leaves to move up to a set distance in a first direction past the first stop, the set distance being less than lengths of the plurality of first beam blocking leaves.

2. The MLC of claim 1, wherein at least one of the plurality of first beam blocking leaves includes a first side and a second side opposing the first side, the first side and the second side extending between the first end and the second end, the first side being configured to contact the first stop when the at least one first beam blocking leaf is moving in the first direction.

3. The MLC of claim 2, wherein the first side includes a first surface and a second surface, the first surface being a greater distance from the second side than the second surface.

4. The MLC of claim 3, wherein the at least one first beam blocking leaf includes a transition portion from the first surface to the second surface and the first stop is configured to contact the transition portion when the first end of the at least one first beam blocking leaf reaches the set distance.

5. The MLC of claim 4, wherein the transition portion extends at angle normal or substantially normal to the first surface.

6. The MLC of one of claims 3 to 5, wherein the first surface and the second surface define a notch therebetween.

7. The MLC of claim 6, wherein the first stop includes at least one elongated member configured to engage the notch.

8. The MLC of claim 7, wherein the at least one elongated member is configured to contact the first surface as the at least one first beam blocking leaf moves.

9. The MLC of one of claims 1 to 8, further comprising:
a second leaf box configured to hold a plurality of second beam blocking leaves, wherein the plurality of second beam blocking leaves are configured to move relative to one another and relative to the second leaf box, each of the plurality of second beam blocking leaves having a first end and a second end; and
a second stop coupled to the second leaf box, the second stop configured to permit the first ends of the plurality of second beam blocking leaves to move up to the set distance past the second stop in a second direction.

10. The MLC of claim 9, wherein the first ends of the plurality of first beam block leaves and the first ends of the plurality of second beam block leaves define at least a portion of the treatment field.

11. A radiation therapy treatment machine comprising:
a radiation source configured to emit a radiation beam; and
a multi-leaf collimator to block portions of the radiation beam to define a treatment field for the radiation therapy treatment machine, the multi-leaf collimator being embodied according to one of claims 1 to 10.
